# EUROPEAN PATENT SPECIFICATION

(11) **EP 4 680 309 B1**
(45) Date of publication and mention of the grant of the patent: **25.03.2026**
(21) Application number: 24726455.9
(22) Date of filing: 19.04.2024
(51) Int. Cl.: A61M 5/315

(54) **FORCE SENSOR FOR MEDICAL DEVICES**
KRAFTSENSOR FÜR MEDIZINISCHE VORRICHTUNGEN
CAPTEUR DE FORCE POUR DISPOSITIFS MÉDICAUX

(30) Priority: 21.04.2023 US 202363461072 P; 02.06.2023 US 202363470684 P; 27.12.2023 US 202318396911
(43) Date of publication of application: 21.01.2026
(73) Proprietor: Flex Ltd., 486123 Singapore (SG)
(72) Inventor: DE ANGELI, Marco, 24030 Barzana (IT); BONGIOVANNI, Antonino, San Jose, California 95002 (US)
(74) Representative: FRKelly
(86) International application number: PCT/US2024/025519
(87) International publication number: WO 2024/220881

(56) References cited:
- WO-A1-2016/162298
- US-A1- 2019 321 555
- US-A1- 2020 206 424

## Description

### FIELD OF THE INVENTION

The present disclosure is directed to disposable syringes, and more particularly it is directed to a disposable syringe including a smart cap with force sensing capabilities.

### BACKGROUND OF THE INVENTION

As is readily known, a syringe is a pumping device generally including a plunger disposed tightly within a cylindrical tube or barrel, and syringes are generally used in the medical industry to inject and dispense a fluid, such as liquid medication. Recently, there is a desire to obtain more data related to medical procedures to improve clinical trials and the overall health of patients. Therefore, smart syringes have been developed which are utilized to collect injection information, provide more refined insights, and perform better statistical analysis of medical trial outcomes. For example, traditional smart syringes can be configured to record one or more of the following: time of injection, duration of injection, dose administered, drug name, expiry date, and other similar data. An issue arises when introducing the smart syringe technology into disposable syringes due to the higher costs associated with smart syringes and the disposable nature of disposable syringes, resulting in an expensive disposable product.

Therefore, there is a need for a cost effective disposable syringe that utilizes traditional smart syringe technology, as well as includes further capabilities to collect data that has not previously been collected using smart syringe technology.

US2020/206424 relates to a smart injection system that includes, among other things, a smart stem that allows for measuring the location of the injection relative to the patient's face and/or the amount of medication injected into the patient. In addition, the smart stem has medication cartridge verification and injector verification features. The smart stem wirelessly transmits the measure data to a processing system. US2019/321555 describes drug delivery systems, devices and methods of use for recording a drug dose completion signal in a data management system. WO2016162298 relates to a portable module for detecting an infusion of the contents of a syringe when the portable module is attached to said syringe.

### SUMMARY OF THE INVENTION

The claimed invention is directed to a syringe including a smart cap as defined in appended claim 1. The detailed description includes the following aspects:
In one aspect, a spring extension of the spring is coupled to a surface of the measuring device facing the plunge end.

In one aspect, the conductive plate is coupled to a surface of the spring facing the battery holder.

In one aspect, the conductive plate is axially offset from the battery holder, with the spacer being positioned between an axial underside of the conductive plate and an axial topside of the battery holder.

In one aspect, an axial underside of the battery holder abuts an axial topside of the battery, and an axial topside of the measuring device abuts an axial underside of the battery.

In one aspect, a battery holder extension of the battery holder is coupled to a surface of the measuring device facing the plunge end.

In one aspect, the measuring device is a printed circuit board.

In one aspect, the printed circuit board is configured to record capacitance measurements and store the capacitance measurements within a memory of the printed circuit board.

According to the invention, the smart cap is configured as a variable capacitor force sensor.

According to the invention, the variable capacitor force sensor is constructed from a first electrode and a second electrode, the first electrode being the conductive plate coupled to the spring and the second electrode being one of the battery or the battery holder.

In one aspect, a force applied to the smart cap causes the plunge rod to translate axially into the barrel, the force causes the first electrode to axially translate towards the second electrode within the cap, and a change in capacitance measured by the measuring device indicates a value of the force applied to the smart cap.

In one aspect, the measuring device, the battery, the battery holder, the spacer, the conductive plate, and the spring are positioned axially in order from the plunge end of the plunge rod in an axial direction extending away from the plunge end and the barrel.

In one aspect, the syringe is a pre-filled disposable syringe.

In one aspect, a light source of the smart cap is configured to illuminate once a compression force applied to the smart cap exceeds a predefined limit.

In one aspect, each of the spring, the conductive plate, the spacer, the battery holder, the battery, and the measuring device are axially aligned with the axis of the plunge rod.

According to the invention, the variable capacitor force sensor includes a first electrode, a second electrode, and a measuring device. The first electrode is a conductive plate coupled to a spring. The second electrode is a battery holder, a battery, or a copper pad. The measuring device can be configured to record capacitance measurements between the first electrode and the second electrode, and the change in capacitance measured by the measuring device indicates a value of the force applied to the variable capacitor force sensor.

In one aspect, the measurement device is a printed circuit board.

In one aspect, the copper pad is coupled to the measuring device.

In one aspect, the variable capacitor force sensor is disposed within a smart cap, and the smart cap is coupled to a plunge end of a plunge rod of a syringe.

### BRIEF DESCRIPTION OF THE DRAWINGS

The foregoing Summary as well as the following Detailed Description will be best understood when read in conjunction with the appended drawings, which illustrate a preferred embodiment of the disclosure. In the drawings:
FIG. 1 is a perspective view of an end portion of a syringe according to the present disclosure.
FIG. 2 is a cross-sectional view of a smart cap of the syringe of FIG. 1.
FIG. 3 is a perspective view of the smart cap with a cap removed, illustrating the components contained within the cap of the smart cap.
FIG. 4 is a perspective view of a glowing light source of the smart cap of FIG. 2.
FIG. 5 is an exploded view of the smart cap, illustrating the individual components of the smart cap.

### DETAILED DESCRIPTION OF THE PREFERRED EMBODIMENTS

Certain terminology is used in the following description for convenience only and is not limiting. The words "front", "rear", "upper", and "lower" designate directions in the drawings to which reference is made. The words "inwardly" and "outwardly" refer to directions towards and away from parts referenced in the drawings. "Axially" refers to a direction along the axis of a shaft, pin, tube, barrel, rod, or the like. A reference to a list of items that are cited as "at least one of a, b, or c" (where a, b, and c represent the items being listed) means any single one of the items a, b, or c, or combinations thereof are included. The terminology includes the words specifically noted above, derivatives thereof, and words of similar import.

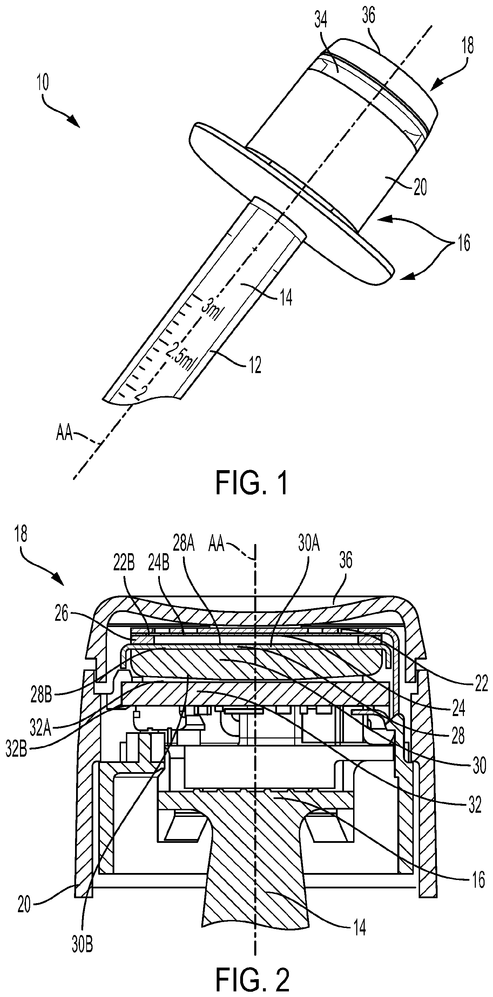

FIG. 1 is a perspective view of an end portion of a syringe 10 according to the present disclosure. The syringe 10 is a pumping device which is generally used in the medical industry to inject and dispense a fluid such as liquid medication, among other options not specifically listed. In some examples, the syringe 10 can be a pre-filled disposable syringe, such that a user receives the syringe pre-filled with a fluid, injects the fluid, and then disposes of (throws away) the pre-filled disposable syringe. In other examples, the syringe 10 may not be a pre-filled or disposable syringe.

The syringe 10 includes a barrel 12, a plunge rod 14, a smart cap 18, and a needle (not shown). The barrel 12 is the body of the syringe 10 that houses the plunge rod 14 and the fluid to be dispensed by the syringe 10. In some examples, as illustrated, the barrel 12 can have a generally elongated cylindrical shape. In other examples, the barrel 12 can have a shape other than an elongated cylindrical shape. Disposed at the end of the barrel 12 not illustrated in FIG. 1, the needle can be coupled to the barrel 12. As is known, the needle is configured to be inserted into the user when dispensing the fluid or medication from within the syringe 10.

The plunge rod 14 is disposed tightly within the barrel 12. Specifically, the plunge rod 14 is axially aligned with the barrel 12, and the plunge rod 14 is axially translatable within the barrel 12 along an axis AA of the plunge rod 14. When pressed and/or squeezed, an end of the plunge rod 14 (not illustrated in FIG. 1) is configured to force the fluid within the barrel 12 out through the needle (not shown). The plunge rod 14 includes a plunge end 16 extending outside the barrel 12 (shown best in FIG. 2). The smart cap 18 is coupled to the plunge end 16 of the plunge rod 14 extending outside the barrel 12. The smart cap 18 is coupled to the plunge end 16 such that the smart cap 18 translates axially along the axis AA with the plunge rod 14. Further, the smart cap 18 is coupled to the plunge end 16 in such a way that when an axial force is applied to the smart cap 18, the smart cap 18 can translate (slightly) axially relative to the plunge end 16, discussed further below. In the illustrated example, the smart cap 18 includes a generally cylindrical shape. In other non-illustrated examples, the smart cap 18 can include any other geometrical shape.

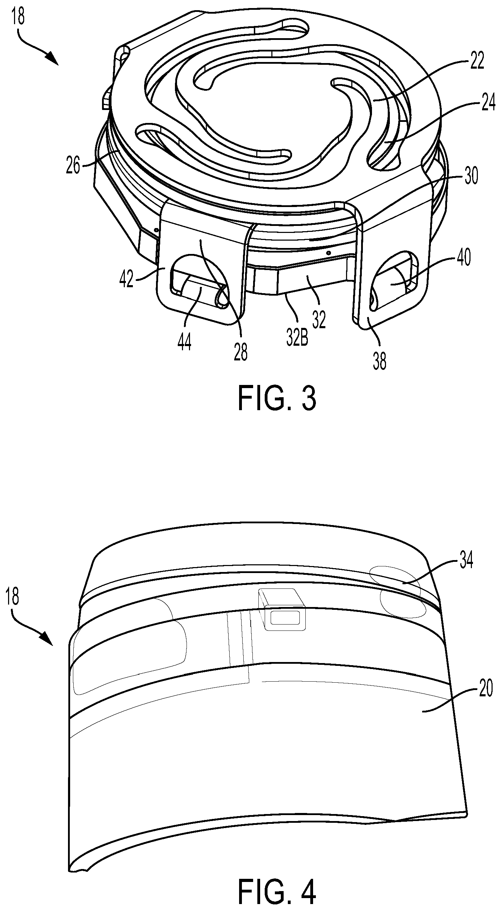

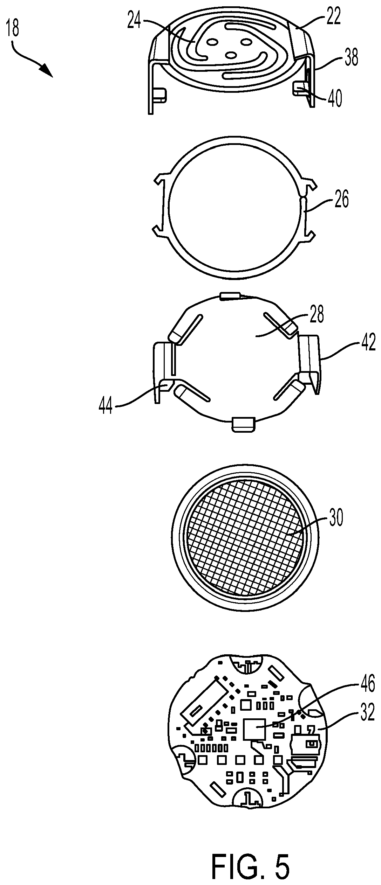

FIG. 2 is a cross-sectional view of the smart cap 18 of the syringe 10. FIG. 3 is a perspective view of the smart cap 18 with a cap 20 of the smart cap 18 removed, illustrating the components contained within the cap 20 of the smart cap 18. FIG. 4 is a perspective view of a glowing light source 34 of the smart cap 18. FIG. 5 is an exploded view of the smart cap 18, illustrating the individual components of the smart cap 18. FIGS. 2-5 will be discussed together.

The smart cap 18 includes a cap 20, a spring 22, a conductive plate 24, a spacer 26, a battery holder 28, a battery 30, a measuring device 32, and a light source 34, among other components not specifically listed. The cap 20 is axially aligned with the plunge rod 14, and the cap 20 encompasses each of the spring 22, the conductive plate 24, the spacer 26, the battery holder 28, the battery 30, the measuring device 32, and the light source 34. The cap 20 is configured to cover and protect each of the components within the smart cap 18 from damage from environmental debris as well as from other sources. The closed end 36 of the cap 20 is positioned furthest from the plunge end 16 of the plunge rod 14. The closed end 36 is the end or surface of the cap 20 that a user presses to cause the plunge rod 14 to translate axially along the axis AA of the plunge rod 14 to dispense or expel the fluid from within the barrel 12 of the syringe 10. In some examples, the cap 20 can be constructed from a polymeric, metallic, or other known material.

As shown best in FIGS. 3 and 5, the spring 22 can be a generally flat circular component with spring extensions 38 extending from the outer circumference of the circular shaped spring 22. The spring extensions 38 extend generally perpendicular to the circular portion of the spring 22, in a direction generally parallel to a central axis of the spring 22. In the illustrated example, the spring 22 includes two spring extensions 38 extending from the spring 22. In other examples, the spring 22 can include more or less than two spring extensions 38 extending from the spring 22. The spring extensions 38 are configured to extend to a lower surface 32B of the measuring device 32, which is positioned closer to the plunge end 16 than the spring 22. Specifically, the spring extensions 38 are configured to be coupled to the lower surface 32B of the measuring device 32 (based on the orientation illustrated in FIGS. 2-3). More specifically, a tab 40 positioned at an end of each of the spring extensions 38 is configured to engage and abut the lower surface 32B of the measuring device 32, coupling the spring 22 to the measuring device 32. The spacer 26 is configured to ensure a gap remains present between the spring 22 and the battery holder 28, discussed further below.

The conductive plate 24 is coupled to the spring 22 such that the conductive plate 24 cannot translate or otherwise move relative to the spring 22. More specifically, the conductive plate 24 is coupled to a lower surface 22B of the spring 22 that faces the plunge end 16 of the plunge rod 14, which lower surface 22B also faces the battery holder 28. The conductive plate 24 is axially offset from the battery holder 28, such that a gap is formed between an axial underside (i.e., a lower surface 24B) of the conductive plate 24 and an axial topside (i.e., an upper surface 28A) of the battery holder 28, with reference to axis AA. More specifically, the spacer 26 is positioned between the conductive plate 24 and the battery holder 28, creating the gap between the conductive plate 24 and the spacer 26. In some examples, the spacer 26 is a washer, such as a plastic washer. In other examples, the spacer 26 can be a washer constructed from a material other than plastic. Further, as illustrated in FIG. 5, in some examples the spacer 26 can have the shape of a generally flat ring. In other examples, the spacer 26 can have a shape other than a generally flat ring. The spacer 26 acts as a fixed thickness washer to control a distance between the conductive plate 24 and the battery holder 28 and/or battery 30 during assembly of the smart cap 18, and at rest when no force is applied to the smart cap 18.

The conductive plate 24 is an electrically conductive component that is constructed from an electrically conductive material, such as, for example, copper or aluminum. As such, in some examples, the conductive plate 24 can be coupled to the spring 22 through various fastening means, such as welding, soldering, brazing, or various adhesives, among other options not specifically listed. In other examples, the conductive plate 24 can be coupled to the spring 22 through mechanical means, such as a fastener, snap-fit connections, or interference fit, among other options not specifically listed. In some examples, the conductive plate 24 can have the shape of a generally flat circular disk. In other examples, the conductive plate 24 can have a shape other than a generally flat circular disk. The conductive plate 24 can be utilized as a first electrode for a variable capacitor force sensor, discussed further below.

As shown best in FIG. 5, the battery holder 28 can have generally the same shape as the spring 22. Specifically, the battery holder 28 can be a generally flat circular component with battery holder extensions 42 extending from the outer circumference of the circular shaped battery holder 28. The battery holder extensions 42 extend generally perpendicular to the circular portion of the battery holder 28, in a direction generally parallel to a central axis of the battery holder 28. In the illustrated example, the battery holder 28 includes two battery holder extensions 42 extending from the battery holder 28. In other examples, the battery holder 28 can include more or less than two battery holder extensions 42 extending from the battery holder 28. The battery holder extensions 42 are configured to extend to the lower surface 32B of the measuring device 32, which is positioned closer to the plunge end 16 than the battery holder 28. Specifically, the battery holder extensions 42 are configured to be coupled to the lower surface 32B of the measuring device 32 (based on the orientation illustrated in FIGS. 2-3). More specifically, a tab 44 positioned at an end of each of the battery holder extensions 42 is configured to engage and abut the lower surface 32B of the measuring device 32, coupling the battery holder 28 to the measuring device 32.

The battery holder 28 is positioned axially closer to the plunge end 16 than the spacer 26, the conductive plate 24, and the spring 22. Further, a lower surface 28B of the battery holder 28 is positioned adjacent and contacts an upper surface 30A of the battery 30. More specifically, an axial underside (lower surface 28B) of the battery holder 28 abuts an axial topside (upper surface 30A) of the battery 30. The battery holder 28 is configured to hold and secure the battery 30 within the cap 20 of the smart cap 18. Further, battery holder 28 is configured to secure the battery 30 to the measuring device 32. The battery holder 28 can be constructed from an electrically conductive material, such as, for example, copper or aluminum. As such, the battery holder 28 and/or the battery 30 can be utilized as a second electrode for a variable capacitor force sensor, discussed further below.

As discussed, the battery 30 can be positioned axially between the battery holder 28 and the measuring device 32. Therefore, an upper surface 32A of the measuring device 32 can be positioned adjacent and abut a lower surface 30B of the battery 30. As illustrated, in some examples, the battery 30 can be a button cell or coin cell battery 30. Therefore, the battery 30 can be a small, circular disc that is configured to store electrical energy. Further, the battery 30 can be configured to supply electrical energy to the smart cap 18 for the smart cap 18 to operate and function. More specifically, the battery 30 can be configured to supply electrical energy to the measuring device 32 and the light source 34, and the battery 30 can be utilized to supply the electrical current utilized to measure the conductance between the first electrode and the second electrode, discussed further below. In some examples, as illustrated, the battery 30 is axially aligned with the axis AA of the plunge rod 14.

The measuring device 32 is disposed axially closer to the plunge end 16 than the battery 30, the battery holder 28, the spacer 26, the conductive plate 24, and the spring 22. In addition, the measuring device 32 is axially aligned with each of the battery 30, the battery holder 28, the spacer 26, the conductive plate 24, and the spring 22, and the measuring device 32 is also axially aligned with the axis AA of the plunge rod 14. In some examples, as illustrated best in FIG. 5, the measuring device 32 can have a generally flat circular shape. In other examples, the measuring device 32 can have a shape other than a generally flat circular shape. The measuring device 32 can be configured to measure and record capacitance measurements, discussed further below.

In some examples, the measuring device 32 can be a printed circuit board that is configured to record capacitance measurements and store the capacitance measurements within a memory 46 of the printed circuit board (measuring device 32). As such, in some examples, the smart cap 18 can be configured as a variable capacitor force sensor. The variable capacitor force sensor can be constructed from a first electrode and a second electrode. In some examples, the first electrode can be the conductive plate 24 coupled to the spring 22, and the second electrode can be one of the battery 30 or the battery holder 28. In other examples, the first electrode can be the conductive plate 24 coupled to the spring 22, and the second electrode can be a conductive copper pad coupled to the upper or topside of the measuring device 32. In either example, the measuring device 32 is configured to measure the capacitance between the first electrode and the second electrode, which can be processed and output as an input force measurement (i.e., force value). Further, in some examples, the capacitance measurements measured by the measuring device 32 can be downloaded to a separate processing device (e.g. a computer) for further processing and analysis.

When a force is applied to the smart cap 18, such as when a user presses and squeezes the closed end 36 of the cap 20 of the smart cap 18 towards the barrel 12, it causes the plunge rod 14 to translate axially into the barrel 12. Further, the pressing or squeezing force on the smart cap 18 causes the first electrode (conductive plate 24) to translate axially towards the second electrode (battery holder 28, battery 30, or copper pad of the measuring device 32) within the cap 20. The measuring device 32 is configured to continuously take capacitance measurements between the first and second electrodes and store the capacitance measurements within the memory 46 of the measuring device 32. Then the measuring device 32 is configured to calculate a change in capacitance measured between the first and second electrodes during the depression of the smart cap 18 and the plunge rod 14 (i.e., during the injection process). The change in capacitance can be computed and converted to identify a change in distance between the first and second electrodes, which can then be computed and converted to identify the compression experienced by the spring 22 (based on the known compression characteristics of the spring 22). Lastly, the compression of the spring 22 can be computed and converted to identify and output the force value applied to the smart cap 18 and the plunge rod 14 during the injection process.

Specifically, a higher compression force leads to a smaller gap between the first and second electrodes, which can be computed and output as a force value by the measuring device 32. A lower compression force leads to a larger gap between the first and second electrodes, which can be computed and output as a force value by the measuring device 32.

It is to be understood that the spring 22 is electrically conductively coupled to the conductive plate 24, and the spring 22 is electrically conductively coupled to the measuring device 32 through the tabs 40 of the spring extensions 38 of the spring 22. Therefore, the spring 22 acts as an electrical conductor and the spring 22 is configured to electrically connect the conductive plate 24 to the measuring device 32 for signal transfer therebetween. In addition, it is to be understood that the battery 30 and the battery holder 28 are each electrically conductively coupled to the measuring device 32 through the tabs 44 of the battery holder extensions 42 of the battery holder 28. Therefore, the battery holder 28 acts as an electrical conductor and the battery holder 28 is configured to electrically connect the battery 30 to the measuring device 32 for signal transfer therebetween.

Lastly, it is to be understood that each of the converting and computing steps described in the previous paragraphs are performed by the measuring device 32 (i.e., the printed circuit board). As such, the measuring device 32 is configured to receive the capacitance measurements, perform converting and computing steps/processes, and then output a force value indicating the force that was applied to the smart cap 18 and the plunge rod 14 during the injection process. The aforementioned information can be utilized to provide more refined insights and perform better statistical analysis of medical trial outcomes. Further, the aforementioned information can be utilized to verify that an air priming has been performed before proceeding with the injection, ensuring safety among other advantages not specifically discussed.

Referring now to FIG. 4, which is a perspective view of the smart cap 18 with the light source 34 glowing or illuminating through the cap 20 of the smart cap 18. The light source 34 can be configured to illuminate once a compression force applied to the smart cap 18 exceeds a predefined limit. As such, the light source 34 can indicate to a user if and when they are applying a force to the smart cap 18 and plunge rod 14 that is greater than the optimal compression force, preventing the user from injecting the fluid within the syringe 10 at a faster than desirable rate. The predefined limit value can vary depending on the fluid or liquid that is within the syringe 10 and being injected into the user. Further, the predefined limit can be any one of a mathematically calculated value, an experimental value, or a theoretical value based on previous data. In some examples, the light source 34 can be a light-emitting diode (LED) that glows or illuminates when electricity is provided to the LED. In other examples, the light source 34 can be a different light source other than an LED that glows or illuminates when electricity is provided to the light source 34. Further, in some examples, the smart cap 18 may not include the light source 34.

The smart cap 18 of the present disclosure provides many advantages that will be appreciated by persons having ordinary skill in the art, compared to previous smart syringe solutions and technologies. Specifically, the smart cap 18 according to the present disclosure improves the cost of the sensing subsystem, making the measurement more affordable and easier to integrate. In turn this creates a new generation of smart syringes that are able to monitor the injection profile over time and complement the data set recorded in real time in clinical trials. Further, the capacitive measurements taken by the smart cap 18 requires less electrical current and energy compared to force sensing resistors (FSR), achieving a longer battery life for the smart cap 18. Additionally, the conductive plate 24 being mechanically connected to the spring 22 allows for an efficient use of the conductive area and improves the linearity and signal to noise ratio of the measuring device 32 and the overall smart cap 18. Also, the use of the battery holder 28 as an electrode reduces the overall part count of the smart cap 18, reducing the cost of material and assembly time for the smart cap 18. The smart cap 18 is a scalable concept that can be applied to other products in the medical industry, as well as other products outside the medical industry, providing a low cost wireless sensing node for force and/or weight measurements.

Overall, the smart cap 18 of the syringe 10 provides a cost reduction for disposable as well as non-disposable smart syringe modules, which is achieved by the disclosed new force sensing solution. In other words, the typical force sensing resistor (FSR) has been replaced with a variable capacitor which relies on cheaper sheet metal parts and leverages the battery holder as a counter electrode, resulting in a smaller, more efficient, and cheaper sensing module/solution. Lastly, the smart cap 18 is smaller, simpler, and easier to assemble (compared to previous smart syringes) due to the miniaturized structure, fewer part count, and ease of assembly, resulting in an overall cost reduction for smart syringe solutions. The aforementioned advantages are only some of the advantages provided by the smart cap 18 and syringe 10 of the present disclosure, as will be appreciated by persons having ordinary skill in the art.

Having thus described the present embodiments in detail, it is to be appreciated and will be apparent to those skilled in the art that many physical changes, only a few of which are exemplified in the detailed description of the disclosure, could be made without altering the inventive concepts and principles embodied therein.

It is also to be appreciated that numerous embodiments incorporating only part of the preferred embodiment are possible which do not alter, with respect to those parts, the inventive concepts and principles embodied therein. The present embodiment and optional configurations are therefore to be considered in all respects as exemplary and/or illustrative and not restrictive, the scope of the disclosure being indicated by the appended claims rather than by the foregoing description.

## Claims

1. A syringe (10) comprising:
a barrel (12) and a plunge rod (14) axially translatable within the barrel (12) along an axis (AA) of the plunge rod (14), the plunge rod (14) including a plunge end (16) extending outside the barrel (12);
a smart cap (18) is coupled to the plunge end (16) of the plunge rod (14) extending outside the barrel (12), **characterised by**
the smart cap (18) comprising:
a spring (22) including a conductive plate (24) coupled to the spring (22);
a spacer (26) disposed axially closer to the plunge end (16) than the spring (22) and the conductive plate (24);
a battery holder (28) disposed axially closer to the plunge end (16) than the spacer (26), the battery holder (28) being adjacent a first surface (30A) of a battery (30);
a measuring device (32) disposed axially closer to the plunge end (16) than the battery holder (28), the measuring device (32) being adjacent a second surface (30B) of the battery (30); and
a cap (20) axially aligned with the plunge rod (14), the cap (20) encompassing each of the spring (22), the conductive plate (24), the spacer (26), the battery holder (28), the battery (30), and the measuring device (32);
wherein the smart cap (18) is configured as a variable capacitor force sensor, and the variable capacitor force sensor is constructed from a first electrode and a second electrode, the first electrode being the conductive plate (24) coupled to the spring (22) and the second electrode being one of the battery (30), the battery holder (28), or a conductive copper pad.

2. The syringe (10) of claim 1, wherein a spring extension (38) of the spring (22) is coupled to a surface (32B) of the measuring device (32) facing the plunge end (16).

3. The syringe (10) of claim 1, wherein the conductive plate (24) is coupled to a surface (22B) of the spring (22) facing the battery holder (28).

4. The syringe (10) of claim 1, wherein the conductive plate (24) is axially offset from the battery holder (28), with the spacer (26) being positioned between an axial underside (24B) of the conductive plate (24) and an axial topside (28A) of the battery holder (28).

5. The syringe (10) of claim 1, wherein an axial underside (28B) of the battery holder (28) abuts an axial topside (30A) of the battery (30), and an axial topside (32A) of the measuring device (32) abuts an axial underside (30B) of the battery (30).

6. The syringe (10) of claim 1, wherein a battery holder extension (42) of the battery holder (28) is coupled to a surface (32B) of the measuring device (32) facing the plunge end (16).

7. The syringe (10) of claim 1, wherein the measuring device (32) is a printed circuit board.

8. The syringe (10) of claim 7, wherein the printed circuit board is configured to record capacitance measurements and store the capacitance measurements within a memory (46) of the printed circuit board.

9. The syringe (10) of claim 1, wherein a force applied to the smart cap (18) causes the plunge rod (14) to translate axially into the barrel (12), the force causes the first electrode to axially translate towards the second electrode within the cap, and a change in capacitance measured by the measuring device (32) indicates a value of the force applied to the smart cap (18).

10. The syringe (10) of claim 1, wherein the measuring device (32), the battery (30), the battery holder (28), the spacer (26), the conductive plate (24), and the spring (22) are positioned axially in order from the plunge end (16) of the plunge rod (14) in an axial direction extending away from the plunge end (16) and the barrel (12).

11. The syringe (10) of claim 1, wherein the syringe is a pre-filled disposable syringe.

12. The syringe (10) of claim 1, further comprising a light source (34) configured to illuminate once a compression force applied to the smart cap (18) exceeds a predefined limit.

13. The syringe (10) of claim 1, wherein each of the spring (22), the conductive plate (24), the spacer (26), the battery holder (28), the battery (30), and the measuring device (32) are axially aligned with the axis of the plunge rod (14).

14. The syringe (10) of claim 1, wherein the measuring device (32) is configured to record capacitance measurements between the first electrode and the second electrode, and a change in capacitance measured by the measuring device (32) indicates a value of the force applied to the variable capacitor force sensor (18).

15. The syringe of claim 1, wherein the copper pad is coupled to the measuring device (32).

## Patentansprüche

1. Spritze (10) umfassend:
einen Körper (12) und eine Tauchkolbenstange (14), die innerhalb des Körpers (12) entlang einer Achse (AA) der Tauchkolbenstange (14) axial verschiebbar ist, wobei die Tauchkolbenstange (14) ein aus dem Körper (12) herausragendes Tauchkolbenende (16) aufweist;
eine intelligente Kappe (18) ist mit dem aus dem Körper (12) herausragenden Tauchkolbenende (16) der Tauchkolbenstange (14) verbunden und zeichnet sich dadurch aus, dass die intelligente Kappe (18) Folgendes umfasst:
eine Feder (22), einschließlich einer leitfähigen Platte (24), die mit der Feder (22) gekoppelt ist;
einen Abstandshalter (26), der axial näher an dem Tauchkolbenende (16) als die Feder (22) und die leitende Platte (24) angeordnet ist;
einen Batteriehalter (28), der axial näher an dem Tauchkolbenende (16) als der Abstandshalter (26) angeordnet ist, wobei der Batteriehalter (28) an eine erste Oberfläche (30A) einer Batterie (30) angrenzt;
eine Messvorrichtung (32), das axial näher am Tauchkolbenende (16) als der Batteriehalter (28) angeordnet ist, wobei die Messvorrichtung (32) an eine zweite Oberfläche (30B) der Batterie (30) angrenzt; und
eine Kappe (20), die axial mit der Tauchkolbenstange (14) ausgerichtet ist und die Feder (22), die leitende Platte (24), den Abstandshalter (26), den Batteriehalter (28), die Batterie (30) und die Messvorrichtung (32) umschließt;
wobei die intelligente Kappe (18) als variabler Kondensatorkraftsensor konfiguriert ist und der variable Kondensatorkraftsensor aus einer ersten Elektrode und einer zweiten Elektrode besteht, wobei die erste Elektrode die mit der Feder (22) gekoppelte leitfähige Platte (24) ist und die zweite Elektrode entweder die Batterie (30), der Batteriehalter (28) oder eine leitfähige Kupferkontaktstelle ist.

2. Spritze (10) nach Anspruch 1, wobei eine Federverlängerung (38) der Feder (22) mit einer Oberfläche (32B) der Messvorrichtung (32) gekoppelt ist, die dem Tauchkolbenende (16) zugewandt ist.

3. Spritze (10) nach Anspruch 1, wobei die leitfähige Platte (24) mit einer Oberfläche (22B) der Feder (22) verbunden ist, die dem Batteriehalter (28) zugewandt ist.

4. Spritze (10) nach Anspruch 1, wobei die leitfähige Platte (24) axial gegenüber dem Batteriehalter (28) versetzt ist und der Abstandshalter (26) zwischen einer axialen Unterseite (24B) der leitfähigen Platte (24) und einer axialen Oberseite (28A) des Batteriehalters (28) angeordnet ist.

5. Spritze (10) nach Anspruch 1, wobei eine axiale Unterseite (28B) des Batteriehalters (28) an eine axiale Oberseite (30A) der Batterie (30) anliegt und eine axiale Oberseite (32A) der Messvorrichtung (32) an eine axiale Unterseite (30B) der Batterie (30) anliegt.

6. Spritze (10) nach Anspruch 1, wobei eine Batteriehalterverlängerung (42) des Batteriehalters (28) mit einer Oberfläche (32B) der Messvorrichtung (32) gekoppelt ist, die dem Tauchkolbenende (16) zugewandt ist.

7. Spritze (10) nach Anspruch 1, wobei die Messvorrichtung (32) eine Leiterplatte ist.

8. Spritze (10) nach Anspruch 7, wobei die Leiterplatte so konfiguriert ist, dass sie Kapazitätsmessungen aufzeichnet und die Kapazitätsmessungen in einem Speicher (46) der Leiterplatte speichert.

9. Spritze (10) nach Anspruch 1, wobei eine auf die intelligente Kappe (18) wirkende Kraft bewirkt, dass sich der Tauchkolbenstange (14) axial in den Körper (12) hineinbewegt, die erste Elektrode axial in Richtung der zweiten Elektrode innerhalb der Kappe verschoben wird und eine von der Messvorrichtung (32) gemessene Kapazitätsänderung den Wert der auf die intelligente Kappe (18) wirkenden Kraft angibt.

10. Spritze (10) nach Anspruch 1, wobei die Messvorrichtung (32), die Batterie (30), der Batteriehalter (28), der Abstandshalter (26), die leitfähige Platte (24) und die Feder (22) axial in der Reihenfolge vom Tauchkolbenende (16) der Tauchkolbenstange (14) in axialer Richtung vom Tauchkolbenende (16) und dem Körper (12) weg angeordnet sind.

11. Spritze (10) nach Anspruch 1, wobei es sich bei der Spritze um eine vorgefüllte Einwegspritze handelt.

12. Spritze (10) nach Anspruch 1, ferner umfassend eine Lichtquelle (34), die so konfiguriert ist, dass sie aufleuchtet, sobald eine auf die intelligente Kappe (18) ausgeübte Kompressionskraft einen vordefinierten Grenzwert überschreitet.

13. Spritze (10) nach Anspruch 1, wobei die Feder (22), die leitfähige Platte (24), der Abstandshalter (26), der Batteriehalter (28), die Batterie (30) und die Messvorrichtung (32) jeweils axial mit der Achse der Tauchkolbenstange (14) ausgerichtet sind.

14. Spritze (10) nach Anspruch 1, wobei die Messvorrichtung (32) so konfiguriert ist, dass sie Kapazitätsmessungen zwischen der ersten Elektrode und der zweiten Elektrode aufzeichnet, und eine von der Messvorrichtung (32) gemessene Änderung der Kapazität einen Wert der auf den variablen Kondenstorkraftsensor (18) ausgeübten Kraft angibt.

15. Spritze nach Anspruch 1, wobei die Kupferkontaktstelle mit der Messvorrichtung (32) gekoppelt ist.

## Revendications

1. Seringue (10) comprenant :
un corps (12) et une tige de piston (14) déplaçable axialement à l'intérieur du corps (12) le long d'un axe (AA) de la tige de piston (14), la tige de piston (14) comportant une extrémité de piston (16) se prolongeant à l'extérieur du corps (12) ;
un capuchon intelligent (18) est couplé à l'extrémité de piston (16) de la tige de piston (14) se prolongeant à l'extérieur du corps (12), **caractérisé par** le capuchon intelligent (18) comprenant :
un ressort (22) comportant une plaque conductrice (24) couplée au ressort (22) ;
une entretoise (26) disposée axialement plus près de l'extrémité de piston (16) que le ressort (22) et la plaque conductrice (24) ;
un porte-batterie (28) disposé axialement plus près de l'extrémité de piston (16) que l'entretoise (26), le porte-batterie (28) étant adjacent à une première surface (30A) d'une batterie (30) ;
un dispositif de mesure (32) disposé axialement plus près de l'extrémité de piston (16) que le porte-batterie (28), le dispositif de mesure (32) étant adjacent à une seconde surface (30B) de la batterie (30) ; et
un capuchon (20) aligné axialement avec la tige de piston (14), le capuchon (20) englobant chacun du ressort (22), de la plaque conductrice (24), de l'entretoise (26), du porte-batterie (28), de la batterie (30) et du dispositif de mesure (32) ;
dans laquelle le capuchon intelligent (18) est configuré comme un capteur de force à condensateur variable, et le capteur de force à condensateur variable est constitué d'une première électrode et d'une seconde électrode, la première électrode étant la plaque conductrice (24) couplée au ressort (22) et la seconde électrode étant l'un de la batterie (30), du porte-batterie (28) ou d'une pastille de cuivre conductrice.

2. Seringue (10) selon la revendication 1, dans laquelle une extension de ressort (38) du ressort (22) est couplée à une surface (32B) du dispositif de mesure (32) faisant face à l'extrémité de piston (16).

3. Seringue (10) selon la revendication 1, dans laquelle la plaque conductrice (24) est couplée à une surface (22B) du ressort (22) faisant face au porte-batterie (28).

4. Seringue (10) selon la revendication 1, dans laquelle la plaque conductrice (24) est décalée axialement par rapport au porte-batterie (28), l'entretoise (26) étant positionnée entre une face inférieure axiale (24B) de la plaque conductrice (24) et une face supérieure axiale (28A) du porte-batterie (28).

5. Seringue (10) selon la revendication 1, dans laquelle une face inférieure axiale (28B) du porte-batterie (28) vient en butée contre une face supérieure axiale (30A) de la batterie (30), et une face supérieure axiale (32A) du dispositif de mesure (32) vient en butée contre une face inférieure axiale (30B) de la batterie (30).

6. Seringue (10) selon la revendication 1, dans laquelle une extension de porte-batterie (42) du porte-batterie (28) est couplée à une surface (32B) du dispositif de mesure (32) faisant face à l'extrémité de piston (16).

7. Seringue (10) selon la revendication 1, dans laquelle le dispositif de mesure (32) est une carte de circuit imprimé.

8. Seringue (10) selon la revendication 7, dans laquelle la carte de circuit imprimé est configurée pour enregistrer des mesures de capacité et stocker les mesures de capacité dans une mémoire (46) de la carte de circuit imprimé.

9. Seringue (10) selon la revendication 1, dans laquelle une force appliquée au capuchon intelligent (18) amène la tige de piston (14) à se déplacer axialement dans le corps (12), la force amène la première électrode à se déplacer axialement vers la seconde électrode à l'intérieur du capuchon, et un changement de capacité mesuré par le dispositif de mesure (32) indique une valeur de la force appliquée au capuchon intelligent (18).

10. Seringue (10) selon la revendication 1, dans laquelle le dispositif de mesure (32), la batterie (30), le porte-batterie (28), l'entretoise (26), la plaque conductrice (24) et le ressort (22) sont positionnés axialement dans l'ordre à partir de l'extrémité de piston (16) de la tige de piston (14) dans une direction axiale se prolongeant à l'opposé de l'extrémité de piston (16) et du corps (12).

11. Seringue (10) selon la revendication 1, dans laquelle la seringue est une seringue jetable pré-remplie.

12. Seringue (10) selon la revendication 1, comprenant également une source lumineuse (34) configurée pour s'illuminer une fois qu'une force de compression appliquée au capuchon intelligent (18) dépasse une limite prédéfinie.

13. Seringue (10) selon la revendication 1, dans laquelle chacun du ressort (22), de la plaque conductrice (24), de l'entretoise (26), du porte-batterie (28), de la batterie (30) et du dispositif de mesure (32) sont alignés axialement avec l'axe de la tige de piston (14).

14. Seringue (10) selon la revendication 1, dans laquelle le dispositif de mesure (32) est configuré pour enregistrer des mesures de capacité entre la première électrode et la seconde électrode, et un changement de capacité mesuré par le dispositif de mesure (32) indique une valeur de la force appliquée au capteur de force à condensateur variable (18).

15. Seringue selon la revendication 1, dans laquelle la pastille de cuivre est couplée au dispositif de mesure (32).
